# EUROPEAN PATENT APPLICATION

(11) **EP 4 316 511 A1**
(43) Date of publication of application: **07.02.2024**
(21) Application number: 22780676.7
(22) Date of filing: 28.03.2022
(51) Int. Cl.: A61K 39/00, A61K 39/12, A61K 39/39, A61K 47/36, A61P 31/12, A61P 31/14, A61P 37/04

(54) **NANOGEL-COATED VACCINE**

(30) Priority: 30.03.2021 JP 2021056392
(71) Applicant: The University of Tokyo, Bunkyo-ku, Tokyo 113-8654 (JP); Hanavax Inc., Chiba-shi, Chiba, 260-0856 (JP)
(72) Inventor: KIYONO Hiroshi, Tokyo 113-8654 (JP); YUKI Yoshikazu, Chiba-shi, Chiba 260-0856 (JP)
(74) Representative: Vossius & Partner Patentanwälte Rechtsanwälte mbB
(86) International application number: PCT/JP2022/014798
(87) International publication number: WO 2022/210465

(57) **Abstract**

It is an object of the present invention to provide: a complex of an antigen that is not encapsulated in a nanogel, and a nanogel; and a vaccine preparation comprising the complex. Specifically, the present invention provides a complex of a nanogel and a vaccine antigen, in which the vaccine antigen is coated with the nanogel

## Description

### Technical Field

The present invention relates to a vaccine in which a nanogel is used. More specifically, the present invention relates to a vaccine that is a complex of a vaccine antigen and a nanogel, in which the antigen is coated with the nanogel.

### Background Art

Conventionally, in most cases, vaccines are administered by injection. Such vaccine administration by injection is capable of inducing immune responses including antibody production *in vivo.* However, it has been difficult for such vaccine administration by injection to induce a protective immune response at the site of viral infection, and as a result, it has been difficult to inhibit the pathogen infection itself. In particular, in the case of viral infection, viruses are often transmitted to other people after infection, but before the onset of symptoms. Accordingly, injectable vaccines could not effectively inhibit viral infection to other people immediately after infection. In contrast to such injectable vaccines, vaccines administered to the mucosa, such as nasal vaccines, can induce mucosal immunity (mucosal IgA) in a mucosal region infected with a pathogen, and can neutralize the pathogen, thereby suppressing infection with the pathogen, and further, the transmission of the pathogen to other people.

To date, in order to effectively induce mucosal immunity, the present inventors have utilized a self-aggregating nano-sized hydrogel composed of cholesterol-loaded cationic pullulan (cationic type of cholesteryl group- bearing pullulan (cCHP)) as a delivery unit for nasal mucosal vaccines (Patent Literature 1 and Non-Patent Literature 1), and have established a method capable of inducing effective mucosal immunity, the method comprising formulating an antigen complex in a form in which a vaccine antigen is encapsulated in a nanogel. When a cCHP nanogel encapsulates a protein antigen in the nanomatrix thereof, it functions as an artificial chaperone, prevents aggregation and degeneration of the antigen, and helps refolding after the release of the antigen. This nanogel has the property of efficiently adhering to the surface of a negatively charged mucosa. The nanogel continuously releases antigens and delivers the antigens to antigen-presenting cells, so as to induce immune response (Non Patent Literature 2, Non Patent Literature 3, and Patent Literature 2). Furthermore, in the case of mice, although a cCHP nanogel that carries [¹¹¹In]-labeled BoHc/A (the heavy chain C-terminal nontoxic region of botulinum toxin type A) or pneumococcal surface antigen PspA is transnasally administered to the mice, it is not accumulated in the central nerve system such as olfactory bulb or brain (Non Patent Literature 2), and the safety thereof has been confirmed (Non Patent Literature 4).

By the way, the previously reported nanogel-antigen complex encapsulates a protein antigen inside a nanogel consisting of about 4 cCHP molecules, thereby preventing aggregation and denaturation of the antigen, promoting the refolding of the antigen released from the inside of the nanogel, and inducing an efficient immune response (Non Patent Literature 5). Therefore, it is impossible to encapsulate molecules having a molecular weight that is larger than the molecular weight of a nanogel (approximately 1,000 kDa) in the nanogel, and thus, it has been thought that such large molecules could not form a complex with a nanogel (Non Patent Literature 5). In other words, the conventional formulation method of encapsulating an antigen in a nanogel could not produce a complex of an antigen such as a viral antigen, VLP (virus like particle) or an inactivated virus, each of which is larger than a nanogel (diameter: about 30 nm), and a nanogel.

### Citation List

### Patent Literature

Patent Literature 1: WO00/12564
Patent Literature 2: JP Patent Publication (Kokai) No. 2010-105968 A

### Non Patent Literature

Non Patent Literature 1: Ayame et al., Bioconjug Chem 19: 882-890, 2008
Non Patent Literature 2: Nochi et al., Nat Mater 9: 572-578, 2010
Non Patent Literature 3: Yuki et al., Biotechnol Genet Eng Rev 29: 61-72, 2013
Non Patent Literature 4: Kong et al., Infect Immun 81: 1625-1634, 2013
Non Patent Literature 5: Yuki et al., Mol. Pharmaceutics, 18: 1582-1592, 2021

### Summary of Invention

### Technical Problem

Considering the aforementioned circumstances, it is an object of the present invention to provide: a complex of an antigen that is not encapsulated in a nanogel (i.e. an antigen exceeding the size that can be encapsulated in a nanogel) and a nanogel; and a vaccine preparation comprising the aforementioned complex.

### Solution to Problem

The present inventors have conducted intensive studies regarding a method of forming a complex of a nanogel and an antigen exceeding the size that can be encapsulated in the nanogel.

The present inventors have attempted to produce a complex, in which a vaccine antigen is not encapsulated in a nanogel but a nanogel is attached to the surface of a vaccine antigen, specifically in which a vaccine antigen is coated with a nanogel (hereinafter, such a complex is also referred to as a "nanogel-coated" complex). As a result, the present inventors have successfully produced a nanogel-coated complex. The present inventors have confirmed that a nanogel-coated antigen induces effective mucosal immunity.

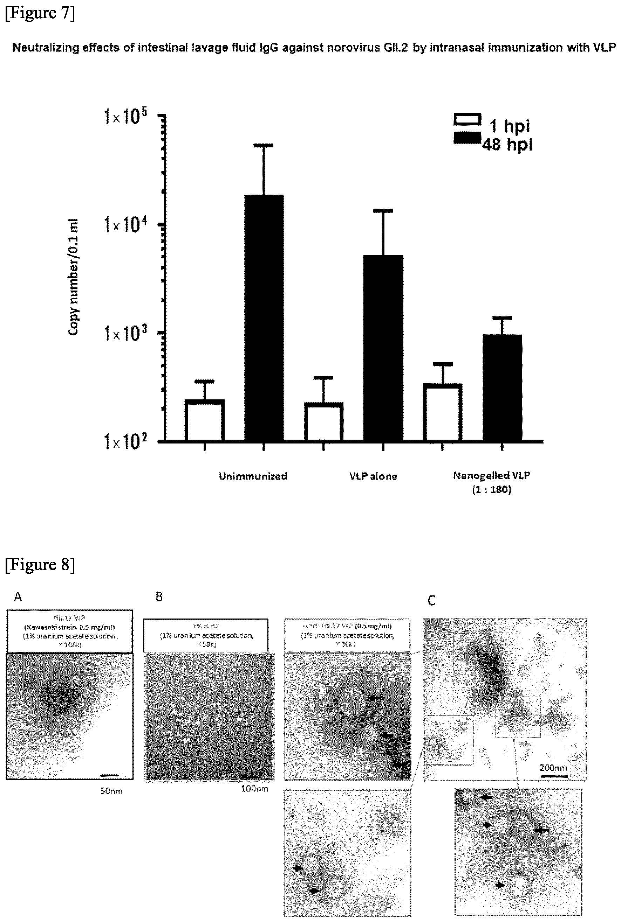

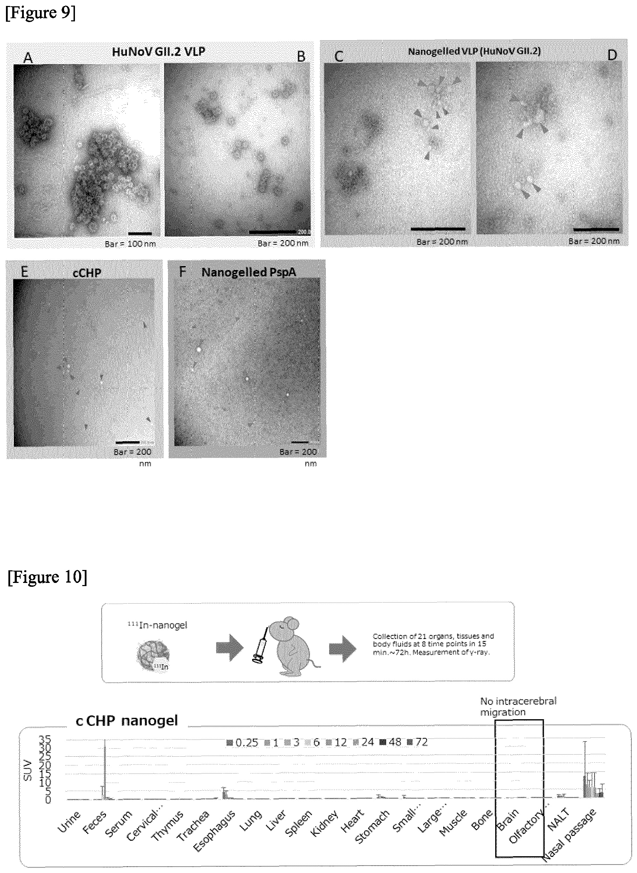

Specifically, the optimal mixing ratio of an antigen and a nanogel, which had been applied for preparation of a conventional antigen-encapsulated nanogel complex (hereinafter also referred to as an "antigen-encapsulating complex"), had been said to be 1 to 2.5 molecules (1 to 10 in CHPNHz equivalent), preferably 1 molecule (4 in CHPNH₂ equivalent) with respect to 1 molecule of the antigen (Patent Literature 2). The present inventors have significantly increased the amount of a nanogel mixed with respect to 1 molecule of norovirus VLP (antigen), and have treated the VLP (antigen) with 18 molecules (72 in CHPNH₂ equivalent) or 180 molecules (720 CHPNH₂ equivalent) of the nanogel. As a result, the present inventors have found for the first time that the VLP (antigen) can be relatively uniformly coated with the nanogel (see Figure 8 and Figure 9). The present inventors have confirmed that transnasal administration of the obtained nanogel-coated VLP to mice can induce a mucosal immune response that is 10 times or more higher than the mucosal immune response obtained by administration of VLP alone. The particle diameter of norovirus VLP is about 30 to 40 nm, and thus, the norovirus VLP is an antigen that exceeds the particle diameter of a nanogel (about 30 nm).

That is to say, the present inventors have found for the first time that even an antigen larger than a nanogel, such as VLP, can form a complex of a nanogel and an antigen complex by coating the antigen with the nanogel (without encapsulating the antigen in the nanogel), and that this nanogel -antigen complex effectively induces mucosal immune response, thereby completing the present invention.

Specifically, the present invention includes the following (1) to (7).
(1) A complex of a nanogel and a vaccine antigen, in which the vaccine antigen is coated with the nanogel.
(2) The complex according to the above (1), which is characterized in that the particle diameter of the vaccine antigen is 20 nm or more.
(3) The complex according to the above (1) or (2), which is characterized in that the vaccine antigen is a substance larger than the nanogel.
(4) The complex according to any one of the above (1) to (3), which is characterized in that the vaccine antigen is VLP (virus like particle), an inactivated virus, a giant protein molecule with a size of 20 nm or more, or a polymer.
(5) The complex according to any one of the above (1) to (4), which is characterized in that the vaccine antigen and the nanogel are complexed with each other at a molar ratio of 1 : 15 to 1 : 200.
(6) The complex according to any one of the above (1) to (5), which is characterized in that it further comprises an adjuvant.
(7) A vaccine preparation, comprising the complex according to any one of the above (1) to (6).

It is to be noted that the preposition "to" sandwiched between numerical values is used in the present description to mean a numerical value range including the numerical values located left and right of the preposition.

### Advantageous Effects of Invention

According to the present invention, since it is possible to form a complex of an antigen and a nanogel although the antigen has a large antigen molecule, an immune response can be effectively induced in the mucosa.

### Brief Description of Drawings

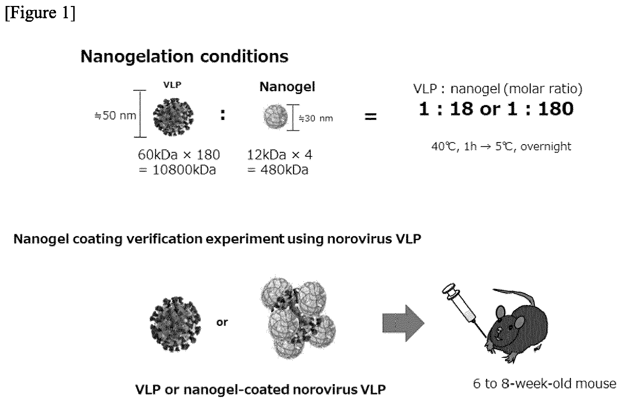

[Figure 1] Figure 1 shows an outline of experimental conditions regarding the nanogel-coated nanogel-antigen complex of the Examples of the present invention.

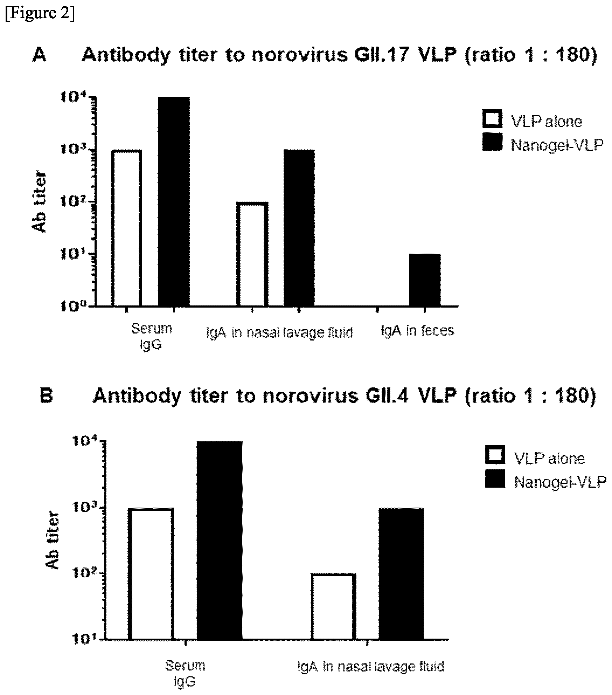

[Figure 2] Figure 2 shows the results of a nasal immune response by nanogel-coated VLP (GII.4 VLP or GII.17 VLP). Figure 2A shows the results obtained by measuring the antibody titers of IgG in serum, IgA in nasal lavage fluid, and IgA in feces, after transnasal administration of nanogel-coated GII.17 VLP or GII.17 VLP alone to mice. Figure 2B shows the results obtained by measuring IgG in serum and IgA in nasal lavage fluid, after transnasal administration of nanogel-coated GII.4 VLP or GII.4 VLP alone to mice. The mixing ratio of the VLP and the nanogel (particles) was set to be VLP : nanogel = 1 : 180 at a molecular ratio.

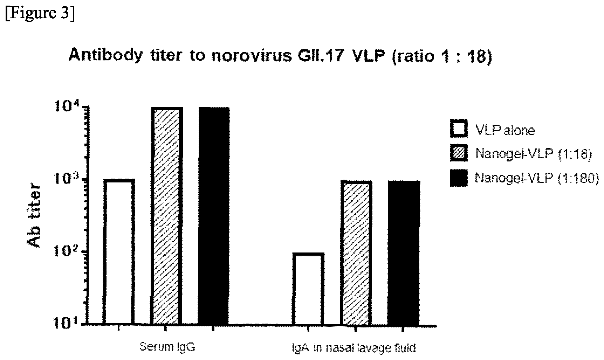

[Figure 3] Figure 3 shows the results of a nasal immune response by nanogel-coated VLP (GII.17 VLP). These are the results obtained by measuring IgG in serum and IgA in nasal lavage fluid, after transnasal administration of nanogel-coated GII.17 VLP or GII.17 VLP alone to mice. The mixing ratio of the VLP and the nanogel (particles) was set to be VLP : nanogel = 1 : 180 or 1 : 18 at a molecular ratio.

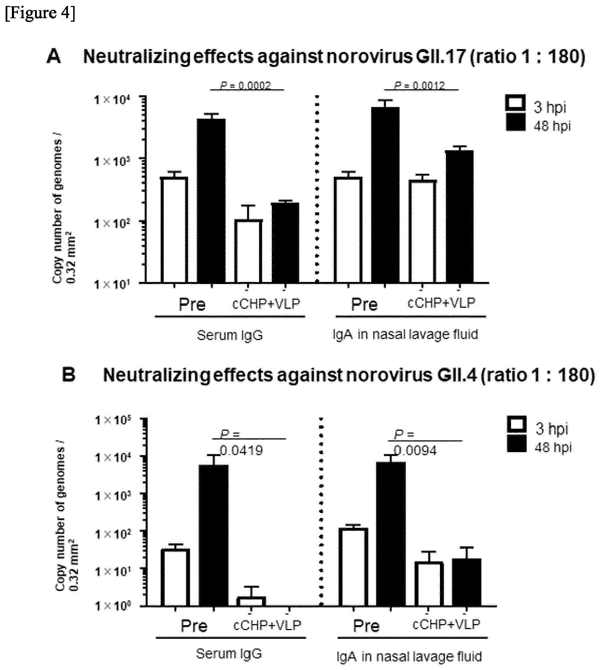

[Figure 4] Figure 4 shows the results obtained by studying the neutralizing effects of antibodies induced by nanogel-coated VLP (GII.4 VLP or GII.17 VLP). Figure 4A shows the results obtained by studying the effect of suppressing proliferation of norovirus GII.17 by IgG in serum and IgA in nasal lavage fluid induced by nanogel-coated GII.17 VLP. Figure 4B shows the results obtained by studying the effect of suppressing proliferation of norovirus GII.4 by IgG in serum and IgA in nasal lavage fluid induced by nanogel-coated GII.4 VLP. "Pre" indicates the results obtained by treating a norovirus solution with mouse serum or nasal lavage fluid before intranasal immunization, then adding the treated norovirus solution to intestinal epithelial cells, and then measuring the copy number of viral genomes in the culture supernatant. "cCHP + VLP" indicates the results obtained by treating a norovirus solution with IgG in serum or IgA in nasal lavage fluid previously induced by nanogel-coated VLP, then adding the treated norovirus solution to intestinal epithelial cells, and then measuring the copy number of viral genomes in the culture supernatant.

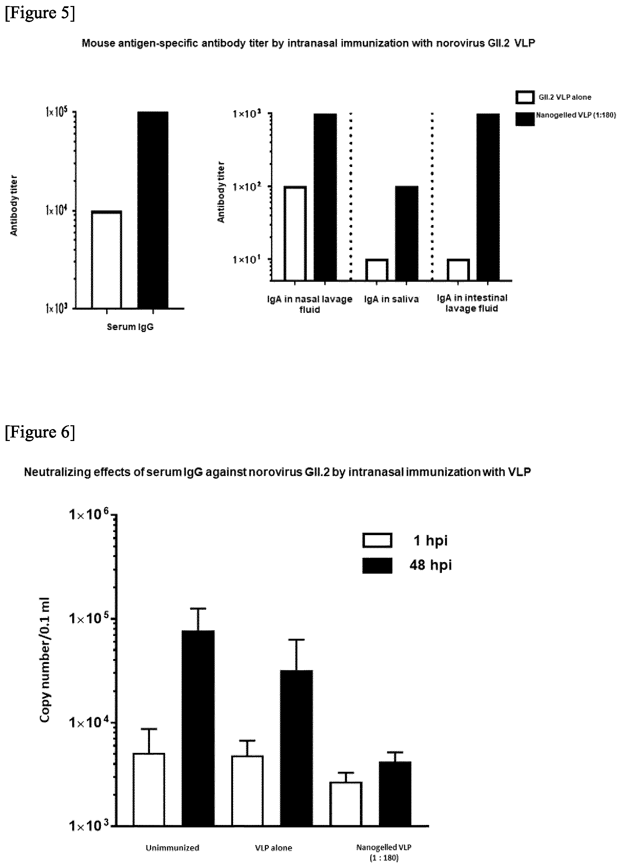

[Figure 5] Figure 5 shows the results of a nasal immune response by nanogel-coated VLP (GII.2 VLP). These are the results obtained by measuring the antibody titers of IgG in serum, IgA in nasal lavage fluid, IgA in saliva, and IgA in intestinal lavage fluid, after transnasal administration of nanogel-coated GII.2 VLP or GII.2 VLP alone to mice. The mixing ratio of the VLP and the nanogel (particles) was set to be VLP : nanogel = 1 : 180 at a molecular ratio.
[Figure 6] Figure 6 shows the results obtained by studying the neutralizing effects of IgG in serum induced by nanogel-coated VLP (GII.2 VLP). These are the results obtained by treating a norovirus solution with mouse serum before intranasal immunization (unimmunized), with IgG in serum induced by VLP alone (VLP alone), or with IgG in serum induced by nanogel-coated VLP (nanogelled VLP), then adding the treated norovirus solution to intestinal epithelial cells, and then measuring the copy number of viral genomes in the culture supernatant.
[Figure 7] Figure 7 shows the results obtained by studying the neutralizing effects of IgA in intestinal lavage fluid induced by nanogel-coated VLP (GII.2 VLP). These are the results obtained by treating a norovirus solution with mouse intestinal lavage fluid before intranasal immunization (unimmunized), with IgA in intestinal lavage fluid induced by VLP alone (VLP alone), or with IgA in intestinal lavage fluid induced by nanogel-coated VLP (nanogelled VLP), then adding the treated norovirus solution to intestinal epithelial cells, and then measuring the copy number of viral genomes in the culture supernatant.
[Figure 8] Figure 8 shows electron microscopic observation images of nanogel-coated VLPs (GII.17 VLPs). Figure 8A is an electron microscopic observation image of GII.17 VLP; Figure 8B is an electron microscopic observation image of a nanogel; and Figure 8C is an electron microscopic observation image of nanogel-coated GII.17 VLP. Figure 8C also shows enlarged images of the areas surrounded with the squares in the observation image.
[Figure 9] Figure 9 shows electron microscopic observation images of nanogel-coated VLP (GII.2 VLP). Figures 9A and B are electron microscopic observation images of GII.2 VLP; Figures C and D are electron microscopic observation images of nanogel-coated GII.2 VLP; Figure 9E is an electron microscopic observation image of a nanogel (cCHP); and Figure 9F is an electron microscopic observation image of a nanogel-PspA complex encapsulating PspA therein.
[Figure 10] Figure 10 shows the results of a mouse pharmacokinetic test involving transnasal administration of an indium (¹¹¹In)-labeled nanogel.

### Description of Embodiments

A first embodiment relates to a complex of a nanogel and a vaccine antigen (hereinafter also referred to as a "nanogel-vaccine antigen (or antigen)"), in which the vaccine antigen is coated with the nanogel (hereinafter also referred to as "the complex according to the present embodiment").

In the present embodiment, the term "nanogel" is used to mean a polymeric gel nanoparticle consisting of a hydrophilic polysaccharide (e.g., pullulan) to which a hydrophobic cholesterol is added as a side chain. Such a nanogel can be produced according to a known method, such as the method described, for example, in International Publication WO00/12564.

Specifically, first, a hydroxyl group-containing hydrocarbon having 12 to 50 carbon atoms or a sterol is allowed to react with a diisocyanate compound represented by OCN-R1 NCO (wherein R₁ represents a hydrocarbon group having 1 to 50 carbon atoms) to produce an isocyanate group-containing hydrophobic compound, with which a single molecule of the hydroxyl group-containing hydrocarbon having 12 to 50 carbon atoms or sterol is reacted. The obtained isocyanate group-containing hydrophobic compound is allowed to react with a polysaccharide to produce a hydrophobic group-containing polysaccharide comprising a hydrocarbon group having 12 to 50 carbon atoms or a steryl group. Subsequently, the obtained product is purified in a ketone-based solvent to produce a hydrophobic group-containing polysaccharide with a high purity.

As a polysaccharide used herein, pullulan, amylopectin, amylose, dextran, hydroxyethyl dextran, mannan, levan, inulin, chitin, chitosan, xyloglucan, watersoluble cellulose, etc. can be utilized, and pullulan is particularly preferable.

Examples of the nanogel used in the present embodiment may include cationic cholesteryl-group-bearing pullulan (referred to as "cCHP") and a derivative thereof. The cCHP has a structure, in which 1 to 10 cholesterols, preferably, 1 to 3 cholesterols are substituted per 100 monosaccharides in pullulan having a molecular weight of 30,000 to 200,000, for example, having a molecular weight of 100,000. Besides, the cCHP used in the present invention may be changed, as appropriate, in terms of the amount of cholesterols substituted, depending on the size of an antigen or the degree of hydrophobicity. In addition, in order to change the degree of hydrophobicity of the CHP, an alkyl group (having approximately 10 to 30, preferably approximately 15 to 20 carbon atoms) may be added to the CHP. The nanogel used in the present invention has a particle diameter of 10 to 50 nm, and preferably of 20 to 30 nm. The nanogels have already been widely marketed, and such commercially available nanogel products may also be used.

The nanogel used in the present embodiment is a nanogel into which a functional group having a positive charge, such as, for example, an amino group, is introduced, so that a vaccine can invade in the surface of the negatively charged nasal mucosa. As a method of introducing an amino group into a nanogel, a method of using an amino group-added cholesterol pullulan (CHPNH₂) can be applied. Specifically, CHP dried under reduced pressure is dissolved in dimethyl sulfoxide (DMSO), and 1,1'-carbonyldiimidazole is then added to the obtained solution under a nitrogen air current, followed by performing a reaction at room temperature for several hours. Thereafter, ethylenediamine is gradually added to the reaction solution, and the obtained mixture is then stirred for several hours to several tens of hours. The obtained reaction solution is dialyzed against distilled water for several days. After completion of the dialysis, the reaction solution is freeze-dried to obtain an opalescent solid. The substitution degree of ethylenediamine can be evaluated using elemental analysis, H-NMR, etc.

The previously reported nanogel-antigen complex has a form in which an antigen is encapsulated in a nanoparticle (nanogel) consisting of about 4 molecules of cationic cholesteryl-group-bearing pullulan (Yuki et al., Molecular Pharmaceutics, https://dx.doi.org/10.1021/acs.molpharmaceut.0c01003 2021). In contrast, the complex according to the present embodiment is a substance in which the size of an antigen exceeds the size that can be encapsulated in nanogel. Therefore, the present complex is characterized by having a form in which a nanogel is added to the surface of an antigen without encapsulation of the antigen in the nanogel (such a form is also referred to as a "form in which an antigen is coated with a nanogel") (see Figure 1, Figure 8, and Figure 9). Herein, the phrase "an antigen that is not encapsulated in a nanogel" is used to mean an antigen having almost the same size as a nanogel, or an antigen larger than a nanogel. The size of the antigen and the size of the nanogel can be compared with each other, for example, according to a method using the particle diameter of the antigen (i.e. a diameter in the case of assuming that the antigen is a sphere) calculated by a light scattering method (e.g. a dynamic light scattering (DLS) method), etc. as an indicator, a method comprising observing the nanogel and the antigen by electron microscopic observation and then evaluating each size, and other methods. However, the comparison methods are not limited to these methods, and those skilled in the art can easily select various methods. More specifically, since the particle diameter of the nanogel is approximately 30 nm (DLS method) (Yuki et al., Molecular Pharmaceutics, https://dx.doi.org/10.1021/acs.molpharmaceut.0c01003 2021), the antigen according to the present embodiment is preferably an antigen in which the particle diameter determined, for example, by the DLS method is approximately 20 nm or more that is slightly smaller than the particle diameter of the nanogel (for example, approximately 20 nm or more and 1,000 nm or less, or approximately 30 nm or more and 600 nm or less). Examples of the antigen according to the present embodiment may include an inactivated virus, VLP (virus like particle), a giant protein molecule with a size of 20 nm or more, and a polymer (for example, a molecule having a molecular weight of 5,000 kDa or more), but the examples of the present antigen are not limited thereto. In addition, the above-described virus may be any virus, and if anything, examples of the virus may include sapovirus, rotavirus, seasonal influenza viruses (type A and B), new (highly virulent) influenza viruses (e.g. H1N1, H5N1, and H7N9 influenza viruses), coronaviruses (e.g. SARS-CoV, SARS-CoV 2, MERS-CoV, etc.), RS viruses (type A and type B), rhinovirus, adenovirus, herpes virus, human papillomavirus, enterovirus, cytomegalovirus, Ebola virus, West Nile virus, Zika virus, Dengue virus, ATL (human adult T-cell virus), HIV, hepatitis A virus, and Chikungunya virus.

The complex according to the present embodiment can be produced by allowing a nanogel and a vaccine antigen to coexist, allowing them to interact with each other, and thereby attaching the nanogel to the surface of the antigen. At this time, the mixing ratio of the nanogel and the vaccine antigen is not particularly limited, and can be easily determined by those skilled in the art according to preliminary experiments. For instance, with regard to the preferred mixing ratio of the vaccine antigen and the nanogel, the mixing ratio can be appropriately selected from the range of the vaccine antigen : the nanogel of, for example, approximately 1 : 10 (40 in CHPNH₂ equivalent) to 1 : 400 (1,600 in CHPNH₂ equivalent), more preferably, approximately 1 : 15 (60 in CHPNH₂ equivalent) to 1 : 200 (800 in CHPNH₂ equivalent 800), at a molar ratio or a molecular ratio.

The complex according to the present embodiment can be produced by mixing a nanogel with a vaccine antigen, and then leaving the obtained mixture at rest at 4°C to 50°C (for example, 40°C) for 30 minutes to 48 hours (for example, about 1 hour). The buffer used to form such a nanogel-vaccine antigen complex is not particularly limited, and if anything, a Tris-HCl buffer or the like can be used.

Moreover, the complex according to the present embodiment may comprise an adjuvant, as well as a nanogel-vaccine antigen complex (the present complex is also included in "the complex according to the present embodiment").

Herein, the term "adjuvant" is synonymous with the term "antigenic reinforcer," "immunostimulant," or the like, and the adjuvant is used for ordinary use purpose of these agents in the present technical field. The active ingredient of the adjuvant used in the present embodiment is not particularly limited, and examples of the active ingredient of the present adjuvant may include STING ligands that activate STING (stimulator of interferon genes) (for example, cyclic dinucleotides such as cGAMP, cyclic-di AMP, cyclic-di GMP, cyclic-di CMP, cyclic-di UMP or cyclic-di IMP, and xanthenone derivatives such as DMXAA (5,6-dimethylXAA (xanthenone-4-acetic acid), Vadimezan or ASA404), polyIC, and CpG ODN. The present adjuvant may further comprise a pharmaceutically acceptable carrier and other components (for example, a stabilizer, a pH adjuster, a preservative, an antiseptic, a buffer agent, etc.). Such a pharmaceutically acceptable carrier and other components need to be substances that do not affect the health of animals to be vaccinated.

When the complex according to the present embodiment comprises an adjuvant, the content of the adjuvant may be approximately 0.01% by weight to 99.99% by weight, based on 100% by weight of a vaccine preparation (see a second embodiment), and may also be, for example, approximately 0.01 weight to 10 weights, based on 1 weight of a vaccine antigen.

The complex of the present embodiment can be formed by mixing a nanogel and a vaccine antigen, or mixing a nanogel, a vaccine antigen and an adjuvant, and then leaving the mixture at rest at a temperature of 4°C to 50°C (e.g. 40°C) for 30 minutes to 48 hours (e.g. about 1 hour). The type of a buffer used in the formation of a complex of a nanogel and a vaccine antigen, or a complex of a nanogel, a vaccine antigen and an adjuvant, is not particularly limited, and if anything, a Tris-HCl buffer may be used, for example.

A second embodiment relates to a vaccine preparation comprising the complex according to the first embodiment, namely, a complex of a nanogel and a vaccine antigen, in which the vaccine antigen is coated with the nanogel (hereinafter also referred to as "the vaccine preparation according to the present embodiment").

When the vaccine preparation according to the present embodiment is used as a composition (the vaccine composition according to the present embodiment), it may comprise pharmaceutically acceptable additives. The vaccine preparation according to the present embodiment is suitable for transnasal administration, and the dosage form of the present vaccine preparation is desirably a form capable of transnasal administration. The vaccine preparation according to the present embodiment may be, for example, a liquid preparation (a nasal drop, an injection, etc.).

When the vaccine preparation according to the present embodiment is a liquid preparation, the active ingredient may be dissolved in distilled water for preparations, as necessary, together with a pH adjuster such as hydrochloric acid, sodium hydroxide, lactose, lactic acid, sodium, sodium monohydrogen phosphate or sodium dihydrogen phosphate, and a tonicity agent such as sodium chloride or glucose, and the obtained solution may be subjected to aseptic filtration and then, the resulting solution may be filled into an ampoule. Otherwise, mannitol, dextrin, cyclodextrin, gelatin or the like may be further added to the resulting solution, followed by vacuum-freeze drying, so as to produce a preparation of extemporaneous dissolution type. The present liquid preparation may comprise pharmaceutically acceptable, known stabilizers, antiseptics, antioxidants, etc. Examples of the stabilizers may include gelatin, dextran, and sorbitol. Examples of the antiseptics may include thimerosal and β propiolactone. An example of the antioxidants may be α tocopherol.

A third embodiment relates to a method for preventing and/or treating a disease, wherein the method comprises transnasal administration of the vaccine preparation according to the second embodiment, namely, a vaccine preparation comprising a complex of a nanogel and a vaccine antigen, in which the vaccine antigen is coated with the nanogel, to a patient.

The target disease of the treatment or prevention of the third embodiment is not particularly limited, and it depends on the type of the vaccine antigen used. The target disease may include cancers as well as infections caused by pathogens.

The vaccine preparation of the present invention may be administered to a patient through the nasal mucosa. The administration method may be, for example, a method of administering the vaccine preparation into the nasal cavity by spraying, coating, dropping or the like of the vaccine preparation onto the nasal mucosa.

The applied dose of the vaccine preparation according to the second embodiment can be determined, as appropriate, depending on the age, body weight and the like of an administration target. The vaccine preparation comprises a pharmaceutically effective amount of vaccine antigen. The pharmaceutically effective amount means the amount of an antigen that is necessary for induction of an immune response to the vaccine antigen. The vaccine preparation may be administered to a target, for example, at a single applied dose of vaccine antigen of several µg to several tens of mg, once to several times per day, with intervals of one week to several weeks, several times in total, for example, 1 to 5 times.

The disclosures of all publications cited in the present description are incorporated herein by reference in their entirety. In addition, throughout the present description, when the description includes singular terms with the articles "a," "an," and "the," these terms include not only single items but also multiple items, unless otherwise clearly specified from the context.

Hereinafter, the present invention will be further described in the following examples. However, these examples are only illustrative examples of the embodiments of the present invention, and thus, are not intended to limit the scope of the present invention.

### Examples

### 1. Materials and methods

### 1-1. Preparation of VLP

Viruses were roughly purified from feces containing HuNoV (human norovirus) furnished from Osaka Institute of Public Health, Osaka, Japan, and a viral genome was then prepared from the viruses. A primer was set outside of the VP1 ORF of GII.4, GII.17, or GII.2 on the prepared genome, and each ORF region was then amplified by PCR, and the nucleotide sequence of the amplified product was then determined. The ORF of each VP1 was cloned into pFastBac Dual Expression Vector (Invitrogen). The amino acid sequence of GII.4 VP1 and a nucleic acid sequence encoding this sequence are shown in SEQ ID NO: 1 and SEQ ID NO: 2, respectively; the amino acid sequence of GII. 17 VP1 and a nucleic acid sequence encoding this sequence are shown in SEQ ID NO: 3 and SEQ ID NO: 4, respectively; and the amino acid sequence of GII.2 VP1 and a nucleic acid sequence encoding this sequence are shown in SEQ ID NO: 5 and SEQ ID NO: 6, respectively. Each construct was used to generate recombinant baculoviruses with the Bac-to-Bac expression system (Invitrogen), after confirming that the sequence thereof was correct. High Five cells (Invitrogen) were infected with each recombinant baculovirus at MOI (multiplicity of infection) and at 7 pfu (plaque-forming units)/cell. Six days after the infection, the culture supernatant was recovered, and was then centrifuged at 20,000 g for 1 hour. The obtained supernatant was ultracentrifuged at 100,000g for 2 hours, and the precipitated VLP was suspended in PBS. The concentrated VLP was then purified by layering on a 10%-60% sucrose density gradient, and then, ultracentrifuging at 100,000g for 1 hour. The VLP passed through the sucrose density gradient was dialyzed against 2 L of PBS three times, so as to remove sucrose in the sample. The VLP was concentrated using an Amicon Ultra 30-kDa centrifugal filter (Millipore).

### 1-2. Coating of norovirus VLP with nanogel

A cCHP nanogel was prepared according to the method of the previous report (Ayame et al., Bioconjugate Chem 19: 882-890, 2008). The prepared nanogel (various calculations were performed, assuming that four molecules of cCHP form one molecule of nanogel; Kuroda et al., Langmuir 18, 3780-3786, 2002) was mixed with the purified norovirus VLP (virus like particles) (various calculations were performed, assuming that 180 molecules of VP1 (60 kDa) form one molecule of VLP; Glass et al., N. Engl. J. Med 361, 1776-1785, 2009) at a molar ratio (VLP : nanogel) of 1 : 18 or 1 : 180. Thereafter, the thus obtained mixture was incubated in a heat block at 40°C for 1 hour, and was then left at rest at 4°C overnight.

### 1-3. Intranasal immunization of mice with nanogel-coated norovirus VLP

A nanogel-VLP complex or VLP (i.e. VLP that does not form a complex; same as below) was administered to 7-week-old female Balb/c mice via transnasal administration. The amount of the antigen administered was set to be 5 µg of VLP protein per animal per administration. Intranasal immunization was performed a total of three times with intervals of 1 week. One week after the final immunization, serum, nasal lavage fluid, intestinal lavage fluid, and feces were collected from the mice. The feces was suspended in 100 mg/mL PBS, and the supernatant was used as a sample.

### 1-4. Measurement of antibody titer of anti-norovirus VLP antibody

Antigen-specific antibody response was analyzed according to an ELISA method (see Kong et al., Infect Immun 81: 1625-1634, 2013). VLP at 1 µg/mL in PBS was coated on a 96-well plate at 4°C overnight. After blocking with PBS-Tween containing 1% BSA, a 10-fold serially diluted sample was added to the resultant, and the obtained mixture was then incubated at room temperature for 2 hours. After washing, HRP-conjugated goat anti-mouse IgG (Southern Biotech) or HRP-conjugated goat anti-mouse IgA (Southern Biotech) was diluted at a ratio of 1 : 4,000, and was then added to the wells, followed by performing incubation at room temperature for 1 hour 30 minutes. After incubation, the sample was chromogenized by TMB Microwell Peroxidase Substrate System (XPL). The endpoint titer was expressed in logarithm (reciprocal log10 titer) for the final dilution factor at which the OD450 value was 0.1 or more higher than that of the negative control.

1-5. Measurement of activity of neutralizing norovirus proliferation, using organoids or monolayered cells induced from human iPS cells

The culture of intestinal organoids induced from a human iPS cell line on Matrigel (Coming), the method of monolayer formation, and the measurement of the neutralizing activity of an anti-VLP antibody against norovirus proliferation using them were performed according to the methods described in a previous report (Sato et al., Cell Mol Gastroenterol Hepatol 7: 686-688, 2019). Norovirus (GII.4_2012 Sydney, GII.17_2015 Kawasaki, and GII.2 OSN201926; furnished from Osaka National Institute of Public Health) solutions were prepared by diluting the viruses with a basic medium (Advanced DMEM/F12 (Gibco) added with 10 mM HEPES (pH 7.3, Gibco ), 2 mM Glutamax (Gibco), and 100 units/mL Penicillin plus 100 µg/mL streptomycin (Gibco)) to 2 x 10⁶ genomic copies per 100 µL. Thereafter, before infection into cells, the diluted virus solutions were each mixed with appropriately diluted mouse antiserum or nasal lavage fluid, or was not mixed with the mouse antiserum or nasal lavage fluid, and was then incubated at 37°C for 90 minutes. The obtained virus solutions were each used to infect intestinal epithelial cells.

The diluted virus solution (100 µL) was added to the monolayered intestinal epithelial cells, so that the intestinal epithelial cells were infected with the virus, and the resulting cells were then incubated at 37°C under 5% CO₂ conditions for 1 hour or 3 hours. Thereafter, the virus solution was removed, and the cells were washed twice with 150 µL of the basic medium. A solution prepared by adding 0.03% bile to a differentiation medium (the basic medium supplemented with a 1x B-27 base medium, 1.25% fetal bovine serum (Biosera), 50 ng/mL mouse EGF, 375 ng/mL mouse R-Spondin 1 (R & D Systems), 50 ng/mL mouse Noggin (Peprotech), and 500 nM A83-01) was added to the wells after washing, and then, the supernatant was immediately recovered. The culture supernatant obtained at 1 hour or 3 hours after the viral infection was used as a 1 hpi (1 hour post infection) or 3 hpi sample, respectively.

Subsequently, 100 µL of the differentiation medium and 0.03% bile were added to the wells after the recovery of the supernatant, and the wells were then incubated at 37°C under 5% CO₂ conditions for 48 hours. Thereafter, this supernatant was recovered as a 48 hpi (48 hour post infection; 48 hours after the infection) sample. With regard to quantification of viral genome copies, RNA was prepared from the diluted virus solution and from the sample recovered at 1 hour (the aforementioned 1 hpi), at 3 hours (the aforementioned 3 hpi) or at 48 hours (the aforementioned 48 hpi) after the infection, using the High Pure Viral RNA Kit (Roche). RT-qPCR was carried out using qPCR (GI/GII) Typing Kit (TakaRa) and StepOne Plus real-time PCR system (Applied Biosystems).

### 1-6. Electron microscopic observation of nanogel-coated norovirus VLP by negative staining

GII.17 VLP and cCHP were subjected to nanogelation at a molar ratio of 1 : 18, or GII.2 VLP and cCHP were subjected to nanogelation at a molar ratio of 1 : 180, so that the obtained products were used as samples for observation. In addition, PspA and cCHP were subjected to nanogelation at a molar ratio of 1 : 1, so as to obtain a previously reported nanogel-encapsulation-type nanogel-antigen complex. GII.17 VLP was diluted with PBS(-) to 0.5 mg/mL, whereas GII.2 VLP was diluted with PBS(-) to 0.1 mg/mL. As cCHP, 1% cCHP was used undiluted. The sample (5 µL) was placed on a grid (MAXTAFORM grid HF36 Cu; 400 mesh) adhered with a carbon-deposited and hydrophilized Formvar support film, and was then stained for 1 minute. The sample solution was removed, and negative staining was performed with a 1% uranium acetate solution (dissolved in distilled water), and the resultant was then observed using a transmission electron microscope (JEM-1400; JEOL Ltd.).

### 2. Results

### 2-1. Outline of the present example

Effective immunity can be induced by encapsulation of an antigen in a nanogel and the subsequent intranasal immunization according to a previously reported method. In this case, the optimal mixing ratio of an antigen and cCHP, which is a monomer, is about 1 : 2 to 1 : 8 at a molecular ratio (antigen : cCHP). When this ratio can be converted to the ratio of an antigen and a spherical nanogel (one molecule of nanogel is formed from four molecules of cCHP: Kuroda et al., Langmuir 18: 3780-3786, 2002), it becomes 1 : 0.5 to 1 : 2 (antigen : nanogel). Accordingly, one molecule of nanogel encapsulates approximately one or two molecules of protein antigens (Yuki et al., Molecular Pharmaceutics, https://dx.doi.org/10.1021/acs.molpharmaceut.0c01003 2021).

With reference to the aforementioned findings, the present inventors have studied whether or not the usefulness of a nanogel for intranasal immunization can be exhibited by coating an antigen with the nanogel (i.e. by attaching the nanogel to the surface of an antigen), when the antigen is larger than the nanogel (i.e., when the antigen cannot be encapsulated in the nanogel). Specifically, norovirus VLPs (GII.17 and GII.4), which form VLPs having a spherical structure with a size of 30 to 40 nm, were used as antigens, and the molecular ratio of a nanogel to the VLP (VLP : nanogel) was set to be about 1 : 180 and 1 : 18. The ratio of the nanogel to the VLP was significantly increased, compared with the conventional method, so as to prepare a nanogel-VLP complex (see Figure 1).

### 2-2. Mouse nasal immune response to nanogel-coated VLPs (GII.17 and GII.4)

The number of nanogel molecules to antigen molecules was set to be a large excess state, namely, the molecular ratio of the nanogel to the norovirus VLP was set to be 1 : 180, and a complex was formed between the norovirus VLPs (GII.17 and GII.4) and the nanogel. Mice were intranasally immunized with GII. 17 VLP alone or with a nanogel-GII. 17 VLP complex three times every other week. The antibody titers of antigen-specific serum IgG, IgA in nasal lavage fluid, and IgA in feces at 1 week after completion of the immunization are shown in Figure 2A. The antibody titers induced in all mice immunized with the nanogel-GII. 17 VLP complex were about 10 times higher than those induced by immunization with the VLP alone.

Figure 2B shows the results of intranasal immunization with GII.4 VLP alone or a nanogel-GII.4 VLP complex under similar conditions. The antibody titers induced in all mice immunized with the nanogel-GII.4 VLP complex were about 10 times higher than those induced by immunization with the VLP alone.

### 2-3. Mouse nasal immune response to nanogel-coated VLP (GII.17)

Subsequently, the mixing ratio of the nanogel to the VLP was set to be 1/10 of the mixing ratio in the above 2-2, namely, the mixing ratio of the nanogel to the antigen (VLP) (VLP : nanogel) was set to be 1 : 18, and a complex was then formed between the norovirus VLP (GII.17) and the nanogel. Mice were intranasally immunized with GII.17 VLP alone or with a nanogel-GII. 17 VLP complex three times every other week. The antibody titers of antigen-specific serum IgG and IgA in nasal lavage fluid at 1 week after completion of the immunization are shown in Figure 3. Even in a case where the mixing ratio of the nanogel to the VLP was decreased, the antibody titers of the antigen-specific serum IgG and the IgA in nasal lavage fluid in all mice immunized with the nanogel-GII.17 VLP complex were about 10 times higher than those induced by immunization with the VLP alone.

### 2-4. Studies regarding neutralizing activity of antibodies induced by nanogel-coated VLPs (GII.17 VLP and GII.4 VLP)

Subsequently, studies were conducted regarding whether or not the antibodies induced by nanogel-coated VLPs (GII.17 VLP and GII.4 VLP) have neutralizing activity, namely, the activity of suppressing proliferation of noroviruses in intestinal epithelial cells. The proliferation of noroviruses was evaluated using the copy number of viral genomes in the culture supernatant as an indicator. The results are shown in Figure 4. It was confirmed that serum IgG and nasal lavage fluid IgA induced by transnasal administration of the nanogel-coated VLPs suppressed the proliferation of norovirus GII.17 and norovirus GII.4 in intestinal epithelial cells, in both cases where the VLP was GII. 17 VLP (Figure 4A) or GII.4 VLP (Figure 4B). In other words, it was found that the antibodies induced by intranasal immunization with the nanogel-coated VLPs have remarkable neutralizing activity.

### 2-5. Mouse nasal immune response to nanogel-coated VLP (GII.2 VLP)

The mixing ratio of the nanogel to GII.2 VLP was set to be 1 : 180, and a complex was formed between the GII.2 VLP and the nanogel. Mice were intranasally immunized with GII.2 VLP alone or with a nanogel-GII.2 VLP complex three times every other week. The antibody titers of antigen-specific serum IgG, IgA in nasal lavage fluid, IgA in saliva, and IgA in intestinal lavage fluid at 1 week after completion of the immunization are shown in Figure 5. The antibody titers in all mice immunized with the nanogel-GII.2 VLP complex were about 10 times to 100 times or more higher than those induced by immunization with the VLP alone. In particular, an increase in the antibody titer in the intestinal lavage fluid was significant.

### 2-6. Studies regarding neutralizing activity of IgG antibody in serum induced by nanogel-coated VLP (GII.2 VLP)

It was confirmed that serum IgG induced by transnasal administration of nanogel-coated VLP more strongly suppresses the proliferation of the norovirus (HuNoV GII. 2) in intestinal epithelial cells, than serum IgG induced by transnasal administration of VLP alone (Figure 6).

### 2-7. Studies regarding neutralizing activity of IgA antibody in intestinal lavage fluid induced by nanogel-coated VLP (GII.2 VLP)

Next, it was confirmed that serum IgG induced by transnasal administration of nanogel-coated VLP more strongly suppresses the proliferation of the norovirus (HuNoV GII. 2) in intestinal epithelial cells, than serum IgA induced by transnasal administration of VLP alone (Figure 7).

It is noteworthy that it could be demonstrated in the present example that intranasal immunization with nanogel-coated GII.2 VLP induce HuNoV GII.2-specific IgA antibodies in the intestinal lavage fluid in an amount 100 times larger than in the case of intranasal immunization with GII.2 VLP alone, and thus that proliferation of HuNoV GII.2 is extremely effectively suppressed. These results demonstrate that clinical application of a nasal vaccine against norovirus is very promising. In other words, it has been previously reported that an adjuvant is needed to obtain the effect of such a nasal VLP vaccine to protect against norovirus (Atma et al., N. Eng. J. Med. 365: 2187-87, 2011). However, taking into consideration the results shown in the present example, it is considered that it is sufficiently possible to produce an adjuvant-free norovirus nasal vaccine by coating an antigen with a nanogel, which is a pharmaceutical additive.

### 2-6. Electron microscopic observation of nanogel-coated VLP by negative staining

Figure 8A shows an electron microscopic observation image of a sample of GII.17 VLP alone. VLP, which is an icosahedral hollow particle with a particle diameter of about 38 nm, was observed. On the other hand, the size (particle diameter) of a nanogel in a sample of 1% cCHP alone was about 20 to 40 nm, and the nanogel was observed as a series of white outlined particles with a slightly weak contrast (Figure 8B). In the electron microscopic observation image of nanogel-coated VLPs, VLPs and nanogels were observed, and also, VLPs forming complexes with nanogels were observed (indicated with the arrows in the enlarged views in Figure 5C). Such VLPs forming complexes with nanogels entirely appeared as white disks because their surfaces were covered with cCHPs (nanogels). In addition, the center of the VLP was concave due to observation under vacuum conditions, and as a result, the VLPs were observed as spherical particles whose contents appeared transparent and slightly dark.

Moreover, Figures 9A and B show observation images of a sample of GII.2 VLP alone. GII.2 VLP, which is an icosahedral hollow particle with a particle diameter of about 30 to 40 nm, was observed. In fact, this GII.2 VLP is formed from 180 VP1 molecules with a molecular weight of 60,000, and the GII.2 VLP has a molecular weight of about 10,000,000. In the observed images of nanogelled GII.2 VLPs (Figures 9C and D), non-nanogelled VLPs and cCHPs are observed, and also, particles that were seemed to be nanogelled are entirely observed as white to light gray three-dimensional spheres because their surfaces are covered with cCHPs (the arrows in the figures). An object having a size slightly larger than VLP was considered to be a coating-state nanogel. This observation is considered to be similar to the aforementioned nanogel-coated GII.17 VLP, the center of which was concave due to observation under vacuum conditions, and which was observed as spherical particles whose hollows appeared transparent and slightly dark. On the other hand, the size of a sample of 1% cCHP alone (Figure 9E) was about 15 to 40 nm, which had a slight variation, and the sample was observed as a white outlined particle. Since it was difficult to confirm PspA alone by transmission electron microscopic observation, nanogelled PspA was observed. As a result, the observation image had almost no significant change (about 20 to 40 nm), compared to the cCHP alone.

The aforementioned electron microscopic observation results suggested that cCHP (nanogel) adheres to the VLP such that it covers the circumference of the VLP, and thereby coats the VLP.

### 2-7. Measurement of particle diameter of nanogel-coated VLP according to DLS (dynamic light scattering) method

In addition to the observation under an electron microscope, the particle diameters of cCHP nanogel, GII.2 VLP, and nanogel-coated GII.2 VLP were measured according to a DLS method, and the measured particle diameters were then compared with one another (Table 1).

**[Table 1]**

| Table 1. DSL Measurement | | |
|---|---|---|
| Sample | D_{H} (nm) | PDI |
| cCHP nanogel | 51.8 | 0.394 |
| GII.2 VLP | 69.7 | 0.345 |
| cCHP nanogelled VLP (molar ratio 1 : 180) | 109.2 | 0.308 |
| cCHP nanogelled PspA (molar ratio 1 : 1) | 53.8 | 0.407 |

As a result, the cCHP nanogel had a particle diameter (DH) of 52 nm and a polydispersity index (PDI) of 0.394. On the other hand, the GII.2 VLP, which had a particle diameter of 70 nm and a PDI value of 0.345, was larger than the TEM observation image thereof. Since the PDI value is slightly large, it is considered that the values of both cases are increased due to the influence of a small amount of aggregates, etc. The nanogel-coated VLP had a particle diameter of 109 nm and a PDI value of 0.308, indicating that the nanogel coating resulted in an increase in the particle diameter. Meanwhile, the particle diameter and PDI of nanogel-encapsulated PspA were almost the same as the particle diameter and PDI of the cCHP nanogel. From the aforementioned results, it was found that the particle diameter of the nanogel-coated VLP antigen was increased, compared with the PspA antigen encapsulated in the nanogel (the nanogel-encapsulated PspA antigen), and the results of the above-described electron microscopic observation (Figure 8 and Figure 9) could be also confirmed from the DSL measurement results.

### 2-8. Studies regarding safety of nanogel (reference)

The nanogel-coated antigen according to the present embodiment (i.e. a complex formed by coating a macromolecular antigen with a nanogel) can be clinically applied as a nasal vaccine without addition of an adjuvant. These results are related to the previous problem regarding failed nasal vaccine development due to intracerebral migration of an influenza nasal vaccine and an *E. coli* heat-labile toxin adjuvant (Mutsch et al., N. Enlg. J Med 350: 896-903, 2004), and thus, these results are considered to be very important. To date, regarding nanogel nasal vaccines, the present inventors have reported the results of pharmacokinetic tests that denied migration of an antigenic portion of the nanogel nasal vaccine into the brain (Yuki et al., J. Immunol. 185: 5436-5443 2010; Fukuyama et al., Mucosal Immunol. 8: 1144-1153 2015). In addition to these results, new data obtained by studying the influence of transnasal administration of a nanogel itself on pharmacokinetics, in particular, on intracerebral migration, are shown below. The results shown in Figure 10 can be considered to be the results of the pharmacokinetic test of a nanogel itself (as a pharmaceutical additive), as well as the results of the pharmacokinetic test of the safety of lipid-based nanoparticles themselves, which are the DDS of a mRNA vaccine (a pharmaceutical additive) given by intramuscular injection.

In order to examine the pharmacokinetics of a nanogel, first, a nanogel was labeled with ¹¹¹In. The ¹¹¹In-labeled nanogel was separated from unreacted ¹¹¹In using a DEAE Sepharose column, and the ¹¹¹In-labeled nanogel was then purified. The purified ¹¹¹In-labeled nanogel (0.1 mg of nanogel) was administered to mice (n = 3) via transnasal administration. Thereafter, 0.25 hours, 1 hour, 3 hours, 6 hours, 12 hours, 24 hours, 48 hours, and 72 hours after the transnasal administration, the radioactivity of ¹¹¹In was measured in various types of organs (Figure 10). As a result, it became clear that the cCHP nanogel as a pharmaceutical additive does not migrate into the olfactory bulb and the brain, and the safely of the nanogel itself could be confirmed. It is to be noted that ¹¹¹In labeling was carried out based on the previous report (Yuki et al., J . Immunol 185: 5436-5443, 2010).

### Industrial Applicability

The present invention provides a nanogel vaccine preparation comprising a large antigen molecule, the formulation of which has been difficult so far, and thus, it is expected that this preparation will be utilized in the medical field.

## Claims

1. A complex of a nanogel and a vaccine antigen, in which the vaccine antigen is coated with the nanogel.

2. The complex according to claim 1, which is **characterized in that** the particle diameter of the vaccine antigen is 20 nm or more.

3. The complex according to claim 1 or claim 2, which is **characterized in that** the vaccine antigen is a substance larger than the nanogel.

4. The complex according to any one of claim 1 to claim 3, which is **characterized in that** the vaccine antigen is VLP (virus like particle), an inactivated virus, a giant protein molecule with a size of 20 nm or more, or a polymer.

5. The complex according to any one of claim 1 to claim 4, which is **characterized in that** the vaccine antigen and the nanogel are complexed with each other at a molar ratio of 1 : 15 to 1 : 200.

6. The complex according to any one of claim 1 to claim 5, which is **characterized in that** it further comprises an adjuvant.

7. A vaccine preparation, comprising the complex according to any one of claim 1 to claim 6.
